(19) Europäisches Patentamt
European Patent Office
Office européen des brevets

(11) Numéro de publication : **0 388 324 B1**

(12) **FASCICULE DE BREVET EUROPEEN**

(45) Date de publication du fascicule du brevet :
**02.09.92 Bulletin 92/36**

(51) Int. Cl.$^5$ : **C07C 15/00,** C07C 2/00

(21) Numéro de dépôt : **90420119.1**

(22) Date de dépôt : **06.03.90**

(54) **Procédé perfectionné pour la préparation d'hydrocarbures aromatiques à partir d'alcanes.**

(30) Priorité : **14.03.89 FR 8903573**

(43) Date de publication de la demande :
**19.09.90 Bulletin 90/38**

(45) Mention de la délivrance du brevet :
**02.09.92 Bulletin 92/36**

(84) Etats contractants désignés :
**DE GB NL**

(56) Documents cités :
**US-A- 4 704 494**

(73) Titulaire : **INSTITUT FRANCAIS DU PETROLE**
**1 Avenue de Bois-Préau**
**F-92500 Rueil Malmaison (FR)**

(72) Inventeur : **Dufaux, Michel**
**13 allée des Pervenches**
**F-69650 St Germain au Mont d'Or (FR)**
Inventeur : **Naccache, Claude Meyer**
**20 rue de Villeneuve**
**F-69130 Ecully (FR)**
Inventeur : **Ben Taarit, Younès**
**106 Chemin de l'Aigas**
**F-69160 Tassin La 1/2 Lune (FR)**

(74) Mandataire : **Laurent, Michel et al**
**Cabinet LAURENT et CHARRAS, 20, rue Louis**
**Chirpaz B.P. 32**
**F-69131 Ecully Cedex (FR)**

EP 0 388 324 B1

## Description

L'invention concerne un procédé perfectionné pour la préparation d'hydrocarbures aromatiques à partir d'alcanes, notamment d'alcanes légers.

La préparation d'hydrocarbures aromatiques à partir d'alcanes est bien connue. Sommairement, cette technique consiste à transformer tout d'abord les alcanes en alcènes par déshydrogénation, puis à oligomériser les alcènes obtenus et enfin à déshydrocycliser ces produits pour obtenir des hydrocarbures aromatiques.

Ces réactions s'effectuent le plus généralement en même temps et en présence de catalyseurs. La transformation est d'autant plus difficile que l'on met en oeuvre des alcanes légers, c'est-à-dire en $C_2$ à $C_5$ (éthane, propane, butane, pentane).

On a suggéré d'effectuer ces réactions en présence de catalyseurs métalliques, tels que Platine, oxyde de Gallium ou oxyde de Vanadium. Malheureusement, ces catalyseurs sont peu actifs et assez peu sélectifs.

On a alors proposé de faire appel à des zéolithes acides type ZSM-5, c'est-à-dire formées d'un aluminosilicate cristallisé de formule :

$$H_7 (Al_7Si_{85}O_{192}).$$

L'emploi de ce catalyseur améliore le rendement et la sélectivité, mais dans des proportions jugées encore trop insuffisantes sur le plan industriel, mais en outre avec une durée de vie trop courte.

Dans les brevets américains US-A-4 120,910, 4,302,620 et 4,350,835, on a suggéré de déposer sur cette zéolithe acide ZSM-5 une petite proportion d'un métal, d'un oxyde métallique ou d'un sel métallique, tel que du Platine, du Gallium, du Plomb, du Zinc, du Tantale. Cette technique ne s'est pas développée commercialement avec succès, car d'une part ces catalyseurs qui restent encore chers se désactivent rapidement (durée de vie inférieure à une heure), et d'autre part sont difficiles à régénérer économiquement.

Dans le brevet américain US-A-4,590,323, on a suggéré d'imprégner la zéolithe acide ZSM-5 d'un sel de vanadium, tel que du sulfate de vanadyle, puis de calciner le produit obtenu à 500°C sous oxygène pour obtenir ainsi un dépôt d'oxyde de vanadium ($V_2O_5$ ) sur la zéolithe. Malheureusement, ce composé se désactive encore plus rapidement que la zéolithe acide ZSM-5 de base et est encore moins actif que celle-ci.

L'invention pallie ces inconvénients. Elle vise un procédé perfectionné du type en question pour la préparation d'hydrocarbures aromatiques à partir d'alcanes, dans lequel on fait appel à un catalyseur meilleur marché, ayant une durée de vie allongée, une meilleure activité et une meilleure sélectivité, un rendement amélioré et qui soit facile à régénérer, du moins de manière économique.

Ce procédé perfectionné pour la préparation d'hydrocarbures aromatiques à partir d'alcanes, par déshydrogénation desdits alcanes, oligomérisation des alcènes obtenus et déshydrocyclisation de ces oligomères, et qui consiste à mettre lesdits alcanes en contact avec un catalyseur constitué par une zéolithe cristalline acide modifiée, se <u>caractérise</u> en ce que ladite zéolithe est un métalloaluminosilicate cristallin de formule :

$$Hx (Al_xM_ySi_{(96-x-y)} O_{192})$$

dans laquelle x est inférieur à 7, y est compris entre 0,01 et 0,5, et dans laquelle M désigne un métal choisi dans le groupe constitué par l'étain, le vanadium et le molybdène.

En d'autres termes, l'invention consiste à mettre en oeuvre comme catalyseur de la réaction, une zéolithe particulière, à savoir un métalloaluminosilicate spécifique et non plus un aluminosilicate recouvert d'oxyde métallique ou de métal. Cette sélection spécifique et étroite dans la grande famille des zéolithes est d'autant plus surprenante que les résultats sont nettement améliorés, tant sur le plan de l'activité, de la sélectivité, que de la durée de vie. En outre et surtout, l'état de la technique (US-A-4,590,323 - sulfate de vanadyle) dissuadait l'homme de métier de faire appel à des composés à base de vanadium, puisque les résultats obtenus étaient jugés trop insuffisants. Bref, l'invention ne découle pas de manière évidente de l'état de la technique, puisque celui-ci éloignait l'homme de métier des composés de vanadium et que la sélection des vanadoaluminosilicates se traduit par des résultats originaux et améliorés.

Ainsi l'invention consiste à utiliser comme catalyseur de la réaction, non plus des aluminosilicates recouverts de métal, mais des aluminosilicates comportant des métaux dans le réseau ou en position intersticielle. Ce changement de structure chimique se traduit de manière surprenante par des résultats nouveaux et nettement améliorés, notamment en ce qui concerne l'activité, la sélectivité et la durée de vie.

Avantageusement, en pratique :

– x est compris entre 4 et 7, et est de préférence voisin de 5 ;

– y est compris de préférence entre 0,05 et 0,1 ;

– M est le vanadium.

x doit être aussi élevé que possible, mais doit permettre de conserver une structure ayant une bonne activité oligomérisante. On a observé que si x était supérieur à 7, le métalloaluminosilicate obtenu n'était pas

2

parfaitement cristallisé, ce qui affecte directement et rapidement l'activité et la durée de vie du catalyseur. On a observé que l'on obtenait les meilleurs résultats lorsque x était voisin de 5.

La présence de vanadium dans la zéolithe contribue à assurer deux fonctions catalytiques distinctes. Le vanadium contribue tout d'abord à assurer la déshydrogénation en alcènes. Il participe également à la modification de la fonction acide qui empêche les réactions parasites de craquage. Lorsque le vanadium est présent, il doit être en quantité limitée. On a ainsi observé que si ce composé était présent à une quantité supérieure à 0,1 %, on augmentait inutilement le coût et on atténuait la fonction acide. Comme déjà dit, on obtient des meilleurs résultats lorsque y est voisin de 0,1 pour le vanadium et de 0,05 pour le molybdène.

Dans une forme d'exécution avantageuse, le catalyseur zéolithique préféré de l'invention répond à la formule :

$$H_{5,3} (Al_{5,3}V_{0,1}Si_{90,6}O_{192})$$

c'est-à-dire dans laquelle x = 5,3, y = 0,1 et M = V.

Dans une variante d'exécution avantageuse sur le plan économique, le catalyseur métalloaluminosilicate mis en oeuvre est régénéré par chauffage progressif jusqu'à 500 à 600°C sous atmosphère d'oxygène.

Les vanadoaluminosilicates caractéristiques de l'invention ont déjà été décrits (DE-A-2 831 631 de BASF). Ces zéolithes ont été proposées pour la transformation du méthanol en essence, pour l'isomérisation du xylène en paraxylène, ou pour l'alkylation du toluène en paraxylène. Il est donc surprenant que le choix de cette zéolithe particulière "dopée" au vanadium permette d'obtenir de telles améliorations dans le procédé de préparation des hydrocarbures aromatiques à partir d'alcanes, alors que, comme déjà dit, l'état de la technique dissuadait de faire appel à des composés de vanadium.

Les métalloaluminosilicates caractéristiques de l'invention sont préparés de manière connue, notamment sous forme de :

$$Rx (Al_xM_ySi_{(96-x-y)}O_{192})$$

dans laquelle R désigne un métal alcalin ou $NH_4$, que l'on active dans le milieu réactionnel pour promouvoir l'activité.

Dans une forme de réalisation pratique, le catalyseur métalloaluminosilicate est préparé à partir d'une zéolithe ZSM - 5.

La manière dont l'invention peut être réalisée et les avantages qui en découlent ressortiront mieux des exemples de réalisation qui suivent.

Le tableau I rassemble les résultats obtenus par les catalyseurs connus de l'état de la technique et cités dans le préambule (exemples 1 à 5).

Le tableau II rassemble les résultats obtenus avec les catalyseurs conformes à l'invention, respectivement :

. vanadoaluminosilicate (exemples 5 à 9),

. molybdoaluminosilicate (exemples 10 et 11),

. stanoaluminosilicate (exemples 12 et 13).

Dans tous les exemples, on place dans un conduit 0,25 gramme de catalyseur à travers lequel passe du propane à pression atmosphérique sous un débit de 0,5 litre/heure. Ce conduit est chauffé dans un four à 550°C. On recueille les produits de la réaction que l'on analyse par chromatographie. A des moments prédéterminés (quinze minutes, une heure, six heures, etc..), on mesure :

– d'une part, le taux global de propane transformé (TTG) ;

– d'autre part, la quantité de composés aromatiques obtenue (sélectivité S).

Exemple 1 :

On utilise comme catalyseur une zéolithe type ZSM-5 de formule :

$$H_7(Al_7Si_{85}O_{192}).$$

de surface spécifique (B.E.T.) 435 m2/g et dans lequel le rapport Si/Al est de 12.

Cette zéolithe très active au démarrage fait preuve d'une excellente sélectivité. En revanche, au bout de quelques heures, elle se désactive très rapidement et sa sélectivité se réduit rapidement (tableau I).

Exemple 2 :

Par les méthodes exposées dans le préambule, on dépose sur la zéolithe de l'exemple 1, 0,35 % de platine par échange puis réduction. Ce catalyseur est plus actif que la zéolithe de base, fait preuve également d'une bonne sélectivité au départ, mais se désactive encore plus rapidement et présente une sélectivité peu différente de celle de la zéolithe de base.

EP 0 388 324 B1

Exemple 3:

On reproduit l'exemple 2 en déposant sur la zéolithe de base ZSM-5, 0,35 % de gallium. On obtient des résultats comparables à ceux de l'exemple 1 avec toutefois une meilleure sélectivité en composés aromatiques.

Exemple 4 :

En reproduisant les enseignements du brevet américain US-A-4 590 323, on dépose sur la zéolithe de base ZSM-5 de l'exemple 1, 0,8 % en poids d'oxyde de vanadium. On obtient une activité et une sélectivité qui est même légèrement inférieure à celle de la zéolithe de base. Cette modification n'améliore donc pas le rendement.

Exemple 5 :

On répète l'exemple 4 en portant la quantité d'oxyde de vanadium à 8 % en poids. Ce catalyseur est moins actif que la zéolithe de base, mais présente la même sélectivité.

En d'autres termes, le dépôt de vanadium sur la zéolithe ZSM-5 ne modifie pas la sélectivité, mais affecte sensiblement l'activité du catalyseur.

Exemple 6 :

On utilise comme catalyseur un vanadoaluminosilicate cristallin de formule :
$$H_{4,0}(Al_{4,0}V_{0,16}Si_{85}O_{192})$$
c'est-à-dire avec x = 4,0, y = 0,16 et M = V.
dans laquelle le rapport Si/Al est de 23 et la surface spécifique (B.E.T.) également de 435 m2/g.

La quantité de vanadium dopé dans la zéolithe est de 0,67 % en poids.

On obtient (voir tableau II) une activité légèrement plus faible que celle de la zéolithe de base, mais soutenue beaucoup plus longtemps. On obtient également une meilleure sélectivité à conversion égale.

En d'autres termes, le catalyseur selon l'invention présente une meilleure sélectivité et surtout une durée de vie plus longue.

Exemple 7 :

On répète l'exemple 6, mais avec un vanadoaluminosilicate de formule :
$$H_{5,3}(Al_{5,3}V_{0,1}Si_{90,7}O_{192}).$$
c'est-à-dire x = 5,3 et y = 0,1.

Cette zéolithe est dopée à raison de 0,42 % en poids de vanadium. Le rapport Si/Al est de 17 et la surface spécifique de 394 m2/g.

On obtient une sélectivité et une activité comparable à celles des catalyseurs de l'état antérieur de la technique, mais on améliore notablement la durée de vie du catalyseur. En d'autres termes, l'incorporation par synthèse directe de vanadium dans la zéolithe de base améliore l'activité, la sélectivité, et surtout la stabilité.

Exemple 8 :

On répète l'exemple 6 avec une zéolithe selon l'invention de formule :
$$H_{6,3}(Al_{6,3}V_{0,08}Si_{89,7}O_{192})$$
c'est-à-dire dans laquelle x = 6,3 et y = 0,08.

Le rapport Si/Al est de 14 et la surface spécifique de 414 m2/g.

La quantité de vanadium dopé par synthèse directe est de 0,32 %.

On obtient une activité plus soutenue, une sélectivité améliorée et une excellente stabilité dans le temps.

Lorsque l'on compare les exemples conformes à l'invention, c'est-à-dire 6, 7 et 8 (tableau II) , on constate qu'une teneur en vanadium supérieure à 0,5 % n'améliore ni l'activité, ni la sélectivité, mais permet toutefois d'obtenir des performances supérieures à celles des catalyseurs de l'art antérieur.

Exemple 9 :

On répète l'exemple 7, mais en régénérant le catalyseur par chauffage progressif depuis la température ambiante jusqu'à 550°C, sous atmosphère d'oxygène, avec une élévation progressive de température de 1°C

4

par minute.

On retrouve les mêmes résultats qu'à l'exemple 7. Cette régénération, qui n'était pas possible jusqu'alors, se traduit donc par une économie appréciable et qui en outre, comme déjà dit, n'affecte pas les résultats.

Exemple 10 :

On répète l'exemple 6 en utilisant comme catalyseur un molybdoaluminosilicate cristallin de formule :

$$H_{5,7} (Al_{5,7}Mo_{0,07}Si_{90,23}O_{192})$$

c'est-à-dire une zéolithe dans laquelle $x = 5{,}7$, $y = 0{,}07$, $M = Mo$, le rapport Si/Al est de 14 et la surface spécifique de 430 m2/g.

La quantité de molybdène dopé dans le réseau de la zéolithe est de 0,11 % en poids.

On obtient (tableau III) des résultats en activité, en sélectivité et en durée de vie, comparables à ceux de l'exemple 8 (V et $y = 0{,}07$).

Exemple 11 :

On répète l'exemple 10 en modifiant la quantité de molybdène, à savoir :

$x = 5{,}3$, $y = 0{,}03$, $M = Mo$ (0,05 % en poids).

Cet abaissement de la quantité de métal dans le réseau de la zéolithe améliore l'activité et la sélectivité. Ainsi, une quantité de molybdène supérieure à 0,05 % n'améliore pas les résultats.

Exemple 12 :

On répète l'exemple 6 en utilisant comme catalyseur un stanoaluminosilicate cristallin de formule :

$$H_{3,6}(Al_{3,6} Sn_{0,16}Si_{92,24}O_{192})$$

c'est-à-dire une zéolithe dans laquelle $x = 3{,}6$, $y = 0{,}16$, $M = Sn$, le rapport Si/al est de 25 et la surface spécifique de 420 m2/g.

On obtient une activité et une sélectivité inférieures à celles des exemples 6 à 11, mais satisfaisantes au plan industriel.

Exemple 13 :

On répète l'exemple 12 avec une zéolithe dopée à l'étain de formule :

$$H_{3,1} (Al_{3,1} Sn_{0,16} Si_{92,74}O_{192})$$

c'est-à-dire $x = 3{,}1$, $y = 0{,}16$ et dans laquelle la surface spécifique est de 360 m2/g.

L'activité et la sélectivité restent comparables à celles de l'exemple 12.

Le procédé selon l'invention présente de nombreux avantages par rapport à ceux connus à ce jour, notamment ceux décrits dans le préambule. On peut citer :
 – la simplicité de mise en oeuvre ;
 – l'amélioration notable de la durée de vie du catalyseur ;
 – l'excellente conservation dans le temps de la sélectivité ;
 – la possibilité de régénération qui n'affecte pas l'activité et la sélectivité, ce qui est original.

De la sorte, le procédé selon l'invention peut être utilisé avec succès pour toutes les synthèses aromatiques à partir d'alcanes, notamment d'alcanes légers.

| EXEMPLES | CARACTERISTIQUES | 15 minutes | 1 heure | 6 heures | 24 heures | 32 heures | 48 heures |
|---|---|---|---|---|---|---|---|
| 1 ZSM$_5$ | TTG | 54 | 53 | 41 | 17 | 16 | 13 |
| | S | 30 | 35 | 32 | 14 | 12 | 10 |
| 2 ZSM$_5$ + 0,35 % Pt dépôt | TTG | 78 | 63,5 | 9 | 3 | - | - |
| | S | 29 | 37 | 21 | 6,5 | - | - |
| 3 ZSM$_5$ + 0,35 % Ga dépôt | TTG | 47 | 50 | 26 | 10 | 8 | 7 |
| | S | 45 | 54 | 51 | 22 | 9 | 9 |
| 4 ZSM$_5$ + 0,8 % V dépôt | TTG | 50 | 52 | - | 15 | 13 | 11 |
| | S | 27 | 33 | - | 14 | 12 | 10 |
| 5 ZSM$_5$ + 8 % V dépôt | TTG | 31 | 25 | 17 | 9 | 9 | 8 |
| | S | 30 | 29 | 34 | 15 | 15 | 11 |

EP 0 388 324 B1

| EXEMPLES | CARACTERISTIQUES | 15 minutes | 1 heure | 6 heures | 24 heures | 32 heures | 48 heures |
|---|---|---|---|---|---|---|---|
| 6<br>ZSM 5 + 0,67 %<br>Vsynth. | TTG | 36 | 36 | 36 | 25 | 22 | 16 |
| | S | 28 | 37 | 32 | 26 | 25 | 20 |
| 7<br>ZSM 5 + 0,42 %<br>Vsynth. | TTG | 50 | 53 | 48 | 28 | 15 | 15 |
| | S | 31 | 36 | 32 | 33 | 24 | 22 |
| 8<br>ZSM 5 + 0,32 % $V_{Synth}$ | TTG | 55 | 49 | 51 | 27 | 16 | 11 |
| | S | 30 | 37 | 39 | 40 | 15 | 7 |
| 9<br>ZSM 5 + 0,42 %<br>Vsynth régénéré | TTG | 48 | – | 56 | 30 | – | 16 |
| | S | 32 | – | 44 | 33 | – | 22 |

7

EP 0 388 324 B1

| EXEMPLES | CARACTERISTIQUES | 15 minutes | 1 heure | 6 heures | 24 heures | 32 heures | 48 heures |
|---|---|---|---|---|---|---|---|
| 10 ZSM 5 + 0,11 % Mo synth. | TTG | – | 40 | – | 35 | 29 | 18 |
| | S | – | 28 | – | 29 | 25 | 18 |
| 11 ZSM 5 + 0,05 % Mo | TTG | – | 47 | 39 | 27 | 24 | 18 |
| | S | – | 32 | 30 | 25 | 23 | 19 |
| 12 ZSM 5 + 1,52 % Sn | TTG | – | 28 | – | 12 | – | – |
| | S | – | 24 | – | 18 | – | – |
| 13 ZSM 5 + 2,31 % Sn | TTG | – | 24 | – | 10 | – | – |
| | S | – | 29 | – | 22 | – | – |

## Revendications

1/ Procédé perfectionné pour la préparation d'hydrocarbures aromatiques à partir d'alcanes, par déshydrogénation desdits alcanes, oligomérisation des alcènes obtenus et déshydrocyclisation de ces oligomères, et qui consiste à mettre lesdits alcanes en contact avec un catalyseur constitué par une zéolithe cristalline acide modifiée,

caractérisé en ce que ladite zéolithe est un métalloaluminosilicate cristallin de formule :

$$Hx\ (Al_xM_ySi_{(96-x-y)}O_{192})$$

dans laquelle x est inférieur à 7, y est compris entre 0,01 et 0,5 et M désigne un métal choisi dans le groupe constitué par l'étain, le molybdène et le vanadium.

2/ Procédé selon la revendication 1, caractérisé en ce que x est compris entre 4 et 7.

3/ Procédé selon la revendication 2, caractérisé en ce que x est voisin de 5.

4/ Procédé selon l'une des revendications 1 à 3, caractérisé en ce que y est compris entre 0,05 et 0,1.

5/ Procédé selon l'une des revendications 1 à 4, caractérisé en ce que le métal M est le vanadium.

6/ Procédé selon la revendication 5, caractérisé en ce que le catalyseur vanadoaluminosilicate répond à la formule :

$$H_{5,3}\ (Al_{5,3}V_{0,1}Si_{90,6}O_{192}).$$

7/ Procédé selon la revendication 1, caractérisé en ce que le catalyseur métalloaluminosilicate est régénéré par chauffage progressif sous atmosphère d'oxygène jusqu'à une température comprise entre 500 et 600°C.

8/ Procédé selon la revendication 1, caractérisé en ce que le catalyseur métalloaluminosilicate est préparé à partir d'une zéolithe ZSM - 5.


## Patentansprüche

1. Verbessertes Verfahren zum Herstellen von aromatischen Kohlenwasserstoffen ausgehend von Alkanen, durch Dehydrieren der Alkane, Oligomerisieren der erhaltenen Alkene und Dehydrocyclisieren dieser Oligomere, bei dem die Alkane mit einem Katalysator in Berührung gebracht werden, der durch einen modifizierten sauren kristallinen Zeolith gebildet ist,

   dadurch gekennzeichnet, daß der Zeolith ein kristallines Metallaluminosilicat der Formel

   $$H_x(Al_xM_ySi_{(96-x-y)}O_{192})$$

   ist, in der x kleiner 7 ist, y zwischen 0,01 und 0,5 liegt, und M ein Metall, ausgewählt aus der Gruppe bestehend aus Zinn, Molybdän und Vanadium, ist.

2. Verfahren nach Anspruch 1, dadurch gekennzeichnet, daß x zwischen 4 und 7 liegt.

3. Verfahren nach Anspruch 2, dadurch gekennzeichnet, daß x in der Nähe von 5 liegt.

4. Verfahren nach einem der Ansprüche 1 bis 3, dadurch gekennzeichnet, daß y zwischen 0,05 und 0,1 liegt.

5. Verfahren nach einem der Ansprüche 1 bis 4, dadurch gekennzeichnet, daß das Metall M Vanadium ist.

6. Verfahren nach Anspruch 5, dadurch gekennzeichnet, daß der Katalysator ein Vanadoaluminosilicat der Formel

   $$H_{5,3}(Al_{5,3}V_{0,1}Si_{90,6}O_{192})$$

   ist.

7. Verfahren nach Anspruch 1, dadurch gekennzeichnet, daß der Metallaluminosilicat-Katalysator durch schrittweises Erwärmen unter Sauerstoffatmosphäre auf eine Temperatur zwischen 500 und 600°C regeneriert wird.

8. Verfahren nach Anspruch 1, dadurch gekennzeichnet, daß der Metallaluminosilicat-Katalysator von einem Zeolith ZSM-5 ausgehend hergestellt wird.

**Claims**

1. An improved process for the preparation of aromatic hydrocarbons from alkanes by dehydrogenation of said alkanes, conversion of the alkenes obtained to the oligomers and dehydro-cyclization of these oligomers, and which consists in bringing said alkanes into contact with a catalyst consisting of a modified acid crystalline zeolite, <u>wherein</u> said zeolite is a crystalline metal aluminosilicate of formula:

$$H_x(Al_xM_ySi_{(96-x-y)}O_{192})$$

in which $\underline{x}$ is less than 7 and $\underline{y}$ is between 0.01 and 0.5 and M denotes a metal chosen from the group comprising tin, molybdenum and vanadium.

2. The process as claimed in claim 1, wherein $\underline{x}$ is between 4 and 7.

3. The process as claimed in claim 2, wherein $\underline{x}$ is close to 5.

4. The process as claimed in one of claims 1 to 3, wherein $\underline{y}$ is between 0.05 and 0.1.

5. The process as claimed in one of claims 1 to 4, wherein the metal M is vanadium.

6. The process as claimed in claim 5, wherein the vanadium aluminosilicate catalyst corresponds to the formula:

$$H_{5.3}(Al_{5.3}V_{0.1}Si_{90.6}O_{192})$$

7. The process as claimed in claim 1, wherein the metal aluminosilicate catalyst is regenerated by progressive heating under an atmosphere of oxygen up to a temperature of between 500 and 600°C.

8. The process as claimed in claim 1, wherein the metal aluminosilicate catalyst is prepared from a ZSM-5 zeolite.